Europäisches Patentamt

European Patent Office

Office européen des brevets

(19)

(11) Publication number: **0 339 567**
**A1**

# EUROPEAN PATENT APPLICATION

(21) Application number: 89107457.7

(22) Date of filing: 25.04.89

(51) Int. Cl.⁴: **C12N 15/00** , **C12P 21/00** , **C12N 1/16** , //(C12N1/16, C12R1:84)

The applicant has filed a statement in accordance with Rule 28 (4) EPC (issue of a sample only to an expert). Accession numbers of the deposits: NRRL Y-15851, NRRL B-18114, NRRL B-15867, NRRL B-15868, NRRL B-15869, NRRL B-15890 and NRRL B-18016.

The microorganisms have been deposited with The Northern Regional Research Center under numbers NRRL Y-15851, B-18114, B-15867, B-15868, B-15869, B-15890 and B-18016.

(30) Priority: 25.04.88 US 186421

(43) Date of publication of application:
02.11.89 Bulletin 89/44

(84) Designated Contracting States:
AT BE CH DE ES FR GB GR IT LI LU NL SE

(71) Applicant: **PHILLIPS PETROLEUM COMPANY**
**5th and Keeler**
**Bartlesville Oklahoma 74004(US)**

(72) Inventor: **Thill, Gregory Patrick**
**3284 W. Fox Run Way**
**San Diego, CA-92111(US)**

(74) Representative: **Dost, Wolfgang, Dr. rer. nat.**
**Dipl.-Chem. et al**
**Patent- u. Rechtsanwälte Bardehle,**
**Pagenberg, Dost, Altenburg Frohwitter &**
**Partner Galileiplatz 1**
**D-8000 München 80(DE)**

(54) Expression of Hepatitis B PreS 2 Protein in Methylotrophic Yeasts.

(57) A process for the enhanced production of antigenic particles consisting essentially of the preS₂ protein. Also disclosed are novel DNA molecules and hosts transformed with these molecules.

FIG. 1

RESTRICTION SITES IN AMG (HBV)

| | |
|---|---|
| Ava I | 1461 |
| Bam HI | 1398 |
| Bam HI | 26 |
| Bgl II | 1982 |
| Bgl II | 2403 |
| Bgl II | 2427 |
| Bst E II | 2819 |
| Dra I | 829 |
| Dra I | 2180 |
| Hae II | 1435 |
| Hinc II | 215 |
| Hinc II | 959 |
| Hinc II | 1680 |
| Hinc II | 2584 |
| Hinc II | 3115 |
| Hpa II | 1303 |
| Hpa II | 1568 |
| Hpa II | 2328 |
| Pst I | 21 |
| Stu I | 965 |
| Stu I | 1110 |
| Stu I | 1697 |
| Xba I | 245 |

EP 0 339 567 A1

## Expression of Hepatitis B PreS$_2$ Protein in Methylotrophic Yeasts

Field of Invention

This invention relates to the field of recombinant DNA biotechnology. In one aspect, this invention relates to a process for the enhanced expression of antigenic particles consisting essentially of the preS$_2$ protein in methylotrophic yeasts. In another aspect the present invention relates to novel DNA molecules and novel yeast strains transformed therewith.

Background

Hepatitis B virus (HBV) causes both acute and chronic diseases and poses a worldwide public health problem. HBV manifests itself as a chronically debilitating infection which can result in progressively severe liver damage, primary carcinoma and death. In the majority of cases, patients completely recover from HBV. However, a significant segment of the population which is infected with HBV becomes chronic carriers of the disease with the potential of transmitting the disease to others.

Recent advances in recombinant DNA techniques have provided many useful methods for elucidating the genetic structure of the HBV, as well as providing the means for preparing vaccines against HBV. The HBV genome is now known to consist of approximately 3.2 kilobase pairs of partially double stranded DNA with a DNA polymerase covalently attached enclosed in a 27nm nucleocapsid. The nucleocapsid is enveloped in a lipoprotein coat consisting of cellular lipids and hepatitis B surface antigens (HBsAg); this is called the virion and is 42nm in diameter.

It has also been discovered that the viral coat consists of three different but related surface proteins. These proteins are referred to generaly as S, PreS$_2$ and PreS$_1$ proteins. Each virion is comprised of 300-400 S protein molecules and 40-80 preS$_2$ and preS$_1$ protein molecules.

The S protein consists of 226 amino acids and is the major component of normal viral lipoprotein coat. The S protein is approximately 24-25 kilodalton (kDa), and may be referred to as P24 or P25. The S protein may also be glycosylated to a 27-28 kilodalton glyco-protein referred to as GP27 or GP28.

The second HBsAg protein is the PreS$_2$ surface antigen, also referred to as the middle HBsAg polypeptide. PreS$_2$ consists of 281 amino acids formed by the addition of 55 amino acids to the N-terminus of the S protein. The PreS$_2$ protein is approximately 31 kilodaltons and may be referred to as the P31 protein. The PreS$_2$ protein also has two glycosylated forms, of 33 kilodaltons and 36 kilodalton, referred to respectively as GP33 and GP36. This antigen is thought to elicit an additional antigenic response in persons who do not respond to S or who respond weakly to S.

The third HBsAg protein is the PreS$_1$ surface antigen, also referred to as the late HBsAg polypeptide. PreS$_1$ consists of between 389-400 amino acids (depending on the antigenic subtype of HBV). The sequence unique to PreS$_1$ consists of 108-119 amino acids which is added to the N-terminus of the complete PreS$_2$ protein. The PreS$_1$ protein is approximately 43 kilodaltons and may also be referred to as the P43 protein. PreS$_1$ also exists in a glycosylated form of 46 kilodaltons designated as GP46 glycoprotein.

In the course of an HBV infection complete viral nucleocapsids are enveloped in a lipoprotein coat, forming 42nm particles. Also formed during the HBV infection are empty 22nm particles which consist mostly of the S and preS$_2$ proteins, and some preS$_1$ proteins. While the complete viral nucleocapsid is infectious, the 22nm empty particles are not infectious. The empty particles, however, will elicit an immune response sufficient to confer immunity and may be used in the preparation of vaccines to HBV.

Hepatitis B vaccines prepared with 22nm particles historically were prepared from the plasma of human carriers of HBV. Unfortunately 22nm particles derived from human plasma must be extensively purified to remove infectious HBV particles as well as any other plasma-borne pathogens. Additionally the preparation of hepatitis B vaccine has been severely restricted because of the limited availability of human plasma.

Utilizing recombinant DNA biotechnology it has been possible to express the hepatitis S protein in a 22nm particle in transformed mammalian cell lines and yeast.

Efforts to produce the antigenic and potentially vaccine-effective PreS$_2$ protein have proven unusually difficult. The PreS$_2$ protein has been found to be very susceptible to proteolysis in recombinant systems. Proteolysis yields two smaller protein fragments which do not retain PreS$_2$'s antigenicity. Additionally, the

PreS2 protein has been very difficult to express in recombinant systems. The expression level of PreS2 is approximately 1/10th the level of the S protein produced in the same recombinant systems.

Thus it would be a significant contribution to the art to develop a process for the production of antigenic HBV particles consisting essentially of unglycosylated PreS2 protein of HBV.

Therefore, it is an object of this invention to provide a process for the enhanced production of antigenic HBV particles consisting essentially of the unglycosylated PreS2 protein of HBV.

Yet another object of this invention is to provide novel vectors containing DNA sequences which code for the PreS2 protein.

A further object of this invention is to provide novel methylotrophic yeasts transformed with a vector or vectors capable of enhanced production of the HBV particle consisting of the PreS2 protein.

Still another object of this invention is the product produced by the process for the production of antigenic HBV particle consisting essentially of unglycosylated PreS2 protein.

These and other objects of the invention will become apparent form the disclosure and claims herein provided.

## Summary of the Invention

In accordance with the present invention, I have discovered process for the enhanced production of an antigenic HBV particle which comprises transforming a methylotrophic yeast with at least one vector having a compatible expression cassette containing a structural gene for PreS2 and culturing the resultant transformants under conditions suitable to obtain the production of particles.

## Detailed Description of the Figures

Figure 1 provides a representation of HBV which contains the PreS2 gene of HBV seratype adw. "Plasmid AM6 is a derivative of the HBV genome shown in Figure 1, wherein the pBR322 plasmid is inserted at the BamHI site at position 26."

Figure 2 provides a representation of plasmid pYM4, a pBR322 derived plasmid containing the Pichia pastoris HIS4 gene inserted at the BamHI site. Brackets indicate that site was destroyed. The HIS4 gene is on deposit within pYJ30 (NRRL B-15890).

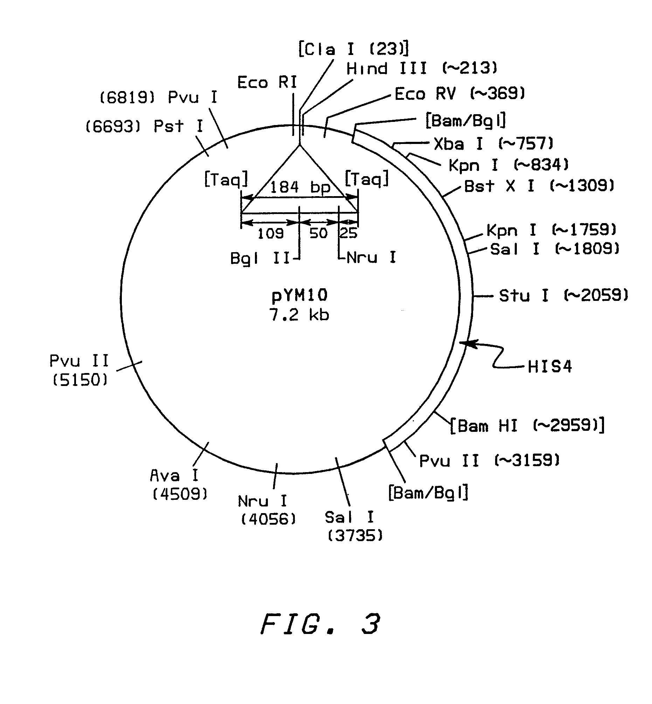

Figure 3 provides a representation of plasmid pYM10. PYM10 is a derivative of pYJ30 (NRRL B-15890) with the BamHI site at 2959 destroyed. The brackets in the Figure indicate a destroyed restriction site.

Figure 4 provides a representation of plasmid pA0804 which contains a linear integrative site-specific vector in the fragment clockwise from BglII toBglII. The structural gene may be inserted in the unique EcoRI site of this plasmid.

## Detailed Description

The PreS2 structural gene is well known in the art and has been sequenced by Lo, Characteristics of PreS2 Region of Hepatitis B Virus, 135 Biochemical and Biophysical Research Communications 382 (1986). Numerous workers in this field have cloned this structural gene and expressed it such as Lo, and Valenzuela, Synthesis and Assembly in Yeast of Hepatitis B Surface Antigen Particles Containing the Polyalbumin Receptor, 3 Biotechnology 317 (1985) to name only two. The structural gene may thus be obtained from workers in the field, synthesized or reisolated. It is also recognized that any PreS2 seratype may be used for the practice of this invention.

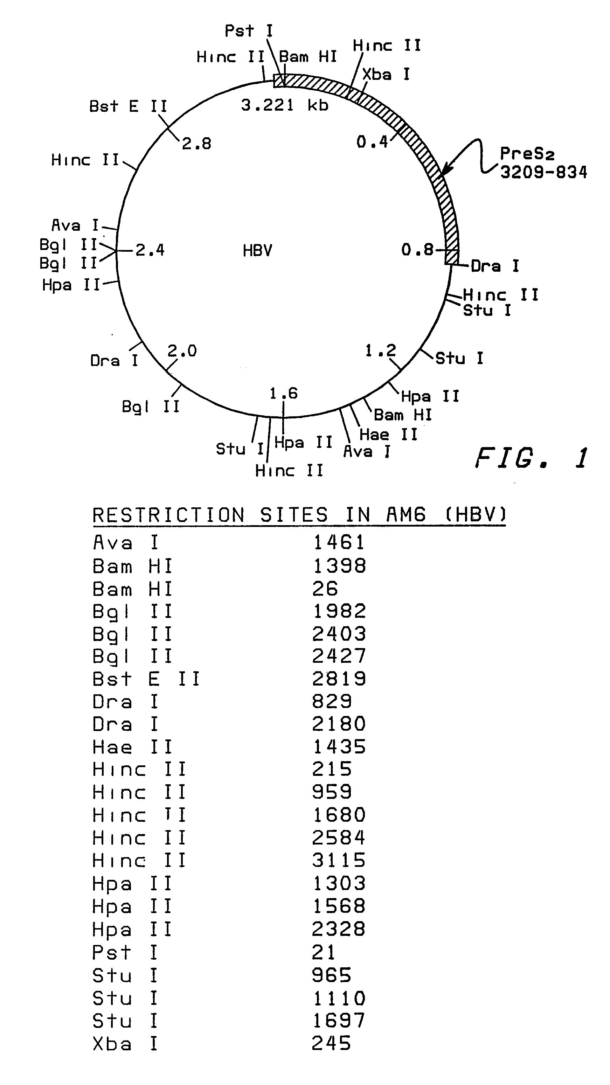

The PreS2 structural gene seratype adw used for the practice of this invention was obtained from plasmid AM6. "Plasmid AM6 is a derivative of the HBV genome shown in Figure 1, wherein the pBR322 plasmid is inserted at the BamHI site at position 26." The nucleotide sequence of this PreS2 structural gene is provided in Table 1. The plasmids used herein may be cultured in any suitable E. coli host such as MC1061.

Two segments of PreS2 structural genes were recovered from plasmid AM6 and the nucleotide

3

sequence for the first 13 amino acids of the N-terminus was synthesized in vitro. The synthesis of the nucleotide sequence used herein can be accomplished by either chemical or enzymatic means, such as chemical procedures based on phosphotriester or phosphite chemistry.

The C-terminus coding region comprising 75% of the structural gene for PreS$_2$ was obtained from plasmid AM6 by a DraI digestion of the plasmid. The DraI digestion was performed using commercially available DraI endonuclease (all endonuclease were used following the manufacturer's recommendation). The DraI fragments were then phenol extracted and ethanol precipitated.

An octameric StuI linker (AAGGCCTT) was then prepared according to standard DNA synthesis techniques and ligated to the DraI fragments using T4 ligase in a blunt end ligation. The ligation was terminated by phenol extraction followed by ethanol precipitation. The resulting fragments were then digested with StuI endonuclease to remove multimers of StuI.

The StuI-linkered fragments were then digested with XbaI. The resultant StuI/XbaI fragment of approximately 600 bp containing the C-terminus coding region of the PreS$_2$ structural gene was isolated by gel electrophoresis.

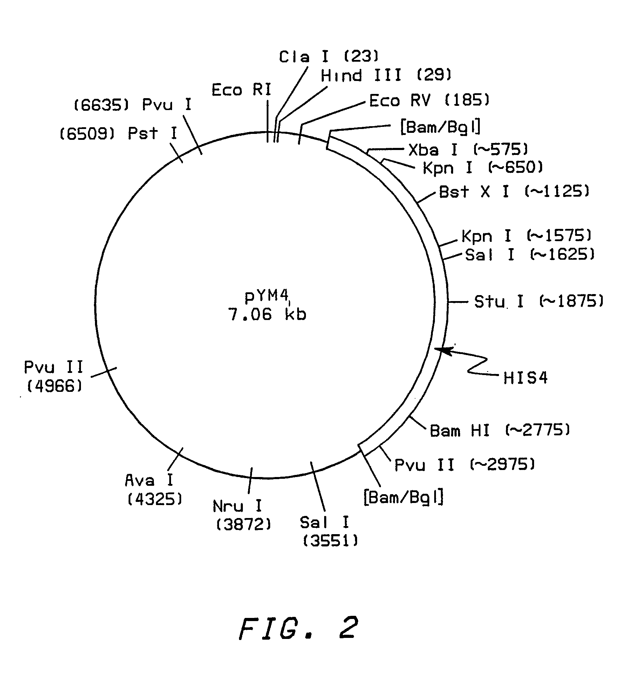

This 600 bp fragment was then ligated with T4 ligase into a 5.7 kb XbaI/Stu digest of plasmid pYM4, Figure 2, which had been isolated by agarose gel electrophoresis.

The ligation mixture was then used directly to transform competent E. coli cells (MC1061), which were then grown in the presence of ampicillin.

## Table 1

```
                7    10              C  20           30              40
G A A T T C A T G C A G T G G A A Ʇ T C C A C T G C C T T C C A C C A A A C T C
        Start of PreS₂

                50              60              70              80
T G C A G G A T C C C A G A G T C A G G G G T C T G T A T C T T C C T G C T G G

                90              100             110             120
T G G C T C C A G T T C A G G A A C A G T A A A C C C T G C T C C G A A T A T T

                130             140             150             160
G C C T C T C A C A T C T C G T C A A T C T C C G C G A G G A C T G G G G A C C

        170 172             180             190             200
C T G T G A C G A A C A T G G A G A A C A T C A C A T C A G G A T T C C T A G G
            Start S

                210             220             230             240
A C C C C T G C T C G T G T T A C A G G C G G G G T T T T T C T T G T T G A C A

                250             260             270             280
A G A A T C C T C A C A A T A C C G C A G A G T C T A G A C T C G T G G T G G A

                290             300             310             320
C T T C T C T C A A T T T T C T A G G G G G A T C T C C C G T G T G T C T T G G

                330             340             350             360
C C A A A A T T C G C A G T C C C C A A C C T C C A A T C A C T C A C C A A C C

                370             380             390             400
T C C T G T C C T C C A A T T T G T C C T G G T T A T C G C T G G A T G T G T C

                410             420             430             440
T G C G G C G T T T A T C A T A T T C C T C T T C A T C C T G C T G C T A T G

                450             460             470             480
C C T C A T C T T C T T A T T G G T T C T T C T G G A T T A T C A A G G T A T G

                490             500             510             520
T T G C C C G T T T G T C C T C T A A T T C C A G G A T C A A C A A C A A C C A

                530             540             550             560
G T A C G G G A C C A T G C A A A A C C T G C A C G A C T C C T G C T C A A G G

                570             580             590             600
C A A C T C T A T G T T T C C C T C A T G T T G C T G T A C A A A A C C T A C G

                610             620             630             640
G A T G G A A A T T G C A C C T G T A T T C C C A T C C C A T C G T C C T G G G
```

Table 1 (Continued)

```
          650              660              670              680
C T T T C G C A A A A T A C C T A T G G G A G T G G G C C T C A G T C C G T T T

          690              700              710              720
C T C T T G G C T C A G T T T A C T A G T G C C A T T T G T T C A G T G G T T C

          730              740              750              760
G T A G G G C T T T C C C C C A C T G T T T G G C T T T C A G C T A T A T G G A

          770              780              790              800
T G A T G T G G T A T T G G G G G C C A A G T C T G T A C A G C A T C G T G A G

          810              820              830              840
T C C C T T T A T A C C G C T G T T A C C A A T T T T C T T T T G T C T C T G G

850 852
G T A T A C A T T T A A A C C C T A A
            stop codon
```

Successfully transformed colonies were selected and the plasmid DNA extracted by the method of Birnboim and Doly [Nucleic Acids Research 7: 1513 (1979)]

The extracted plasmid DNA was digested with StuI, phenol extracted and ethanol precipitated. EcoRI linkers were prepared and T4 ligated to the StuI fragments. Excess linkers were removed by EcoRI digestion. These EcoRI linkered fragments were further digested with XbaI and electrophoresised to isolate the fragments containing the C-terminal portion of the preS2 structural gene.

The XbaI-EcoRI digest was ligated into XbaI-EcoRI-cut pUC18. The ligation mixture was transformed into competent E. coli cells (MC1061), which was then grown in the presence of ampicillin. Transformed cells containing the C-terminal portion of the preS2 structural gene were selected by fragment digestion analysis employing ClaI and XbaI. The plasmid selected by this process was designated pHS2-B.

The middle portion of the preS2 gene was recovered by first digesting plasmid AM6 with XbaI and BamHI. The desired 250 bp fragment was isolated by gel electrophoresis. The 250 bp XbaI/BamHI fragment was then ligated into pUC18 which had been digested with XbaI and BamHI and used to transform E. coli MC1061. Cultures which grew in the presence of ampicillin were analyzed by recovering plasmid DNA and digesting it with BamHI. Those plasmids which contained a 2.7 Kb linear fragment upon electrophoresis were deemed to have the desired 250 bp fragment. One transformed colony containing the 250 bp fragment was isolated and grown on a large scale. The plasmid DNA was isolated and purified from the colony which was digested with EcoRI and BamHI. The vector band was isolated by electrophoresis. The vector was then ligated with the following kinased double stranded oligonucleotide.

```
        PstI    BamHI

5' AATTCAATCCGTCTGCAG      3'

        3'GTTAGGCAGACGTCCTAG   5'
```

The ligation reaction mixture was used to transform E. coli MC1061 and colonies with the correct insert selected by ampicillin resistance. This plasmid was designated pPS2.

The N-terminus coding region of preS2 was prepared as a synthetic oligonucleotide of the following sequence.

```
     HindIII                                        PstI

5' AGCTTGAATTCATGCAGTGGAACTCCACTGCCTTCCACCAAACTCTGCA 3'

     ACTTAAGTACGTCACCTTGAGGTGACGGAAGGTGGTTTGAG 5'
```

This sequence was cloned into a HindIII and PstI digest of pUC18. The desired transformants were

6

characterized by the presence of an approximately 75 bp fragment after EcoRI digestion and electrophoresis. The plasmids selected by the this method were designated pTBO-2A.

The middle portion of the gene was added by digesting the vector pTBO-2A with PstI and XbaI; the insert was the 250 bp PstI-XbaI fragment from pPS2. Transformants were characterized by the presence of a >300 bp EcoRI fragment as well as 290 bp XbaI/HindIII fragment. The correct isolate was termed pTBO-3. The complete preS2 gene was achieved by inserting the HindIII/XbaI fragment from pTBO-3 into XbaI/HindIII-digested pHS2B. Ampicillin resistant transformants were characterized by the presence of a 825 bp EcoRI fragment. This construct was termed pTBO4.

Culturing the E. coli strain listed above may be accomplished by any suitable means. General techniques for culturing E. coli are already known in the art and any adaptation of these methods to the specific requirements of the strains used herein is well within the abilities of those skilled in the art.

Recovery of plasmid DNA from E. coli can be accomplished by several techniques due to its compact size and closed spherical superhelical form. For example following the harvest, host cells may be pelleted by centrifugation and then resuspended and lysed. The lysate should be centrifuged to remove cell debris and the supernatant containing DNA retained. A phenol extraction can then be performed to remove most other contaminants from the DNA. The phenol-extracted DNA may then be further treated using a density gradient centrifugation or a gel filtration technique to separate the plasmid DNA from the bacterial DNA. The techniques for achieving the separation alluded to above are well known in the art and numerous methods of performing these techniques are known.

Nuclease digestion of the plasmid may be accomplished by choosing appropriate endonucleases which will cut the selected plasmid in such a way as to facilitate the recovery of the preS2 structural gene. The endonucleases used will depend on the plasmid from which the preS2 gene is to be excised. For example, the preS2 structural gene contained in plasmid AM6 could be recovered in a DraI-HincII fragment.

Gel electrophoresis of DNA may be accomplished using numerous techniques. See P. G. Sealy and E. M. Southern, Gel Electrophoresis of Nucleic Acids - A practical Approach (D. Rickwood and B. D. Hames, eds.) p. 39 (1982). Elution may also be accomplished using numerous techniques appropriate for the gel involved, such as electroelution, diffusion, gel dissolution (agarose gels) or physical extrusion (agarose gels). It is additionally recognized that elution may not be necessary with some gels such as high-quality, low melting temperature agarose.

Once the fragment containing the preS2 structural gene or fragments thereof is isolated, additional manipulations may be required before it is inserted in the vector. These manipulations may include, but are not limited to the addition of linkers or blunt-ending the fragment.

Following the isolation of the preS2 structural gene, the gene is inserted into a suitable methylotrophic yeast vector such as a plasmid. Preferable vectors for the practice of this invention are those compatible with the Pichia genus and most preferably Pichia pastoris.

Plasmids have long been one of the basic elements employed in recombinant DNA technology. Plasmids are circular extrachromosomal double-stranded DNA found in microorganisms. Plasmids have been found to occur in single or multiple copies per cell. Included in plasmid DNA is the information required for plasmid reproduction, i.e. an origin of replication is included for bacterial replication. One or more means of phenotypically selecting the plasmid in transformed cells may also be included in the information encoded in the plasmid. Phenotypic or selection markers, such as antibiotic resistance genes or genes which complement defects in the host biochemical pathways, permit clones of the host cells which have been transformed to be recognized, selected, and maintained.

To express the preS2 structural gene in methylotrophic yeast, the gene must be operably linked to a 5' regulatory region and 3' termination sequence, which forms the expression cassette which will be inserted into the host via a vector.

The following terms are defined herein for the purpose of clarification.

Operably linked--refers to a juxtaposition wherein the components are configured so as to perform their function.

Regulatory region--DNA sequences which respond to various stimuli and affect the rate of mRNA transcription.

3' Termination sequence--sequences 3' to the stop codon which function to stabilize the mRNA such as sequences which elicit polyadenylation.

"Pichia compatible" refers to DNA sequences which will perform their normal function in Pichia such as regulatory regions and 3' termination sequences derived from Pichia.

Preferred for the practice of the present invention are integrative vectors, such as the linear site-specific integrative vector of Cregg, as described in European Application Serial Number 86114700.7. Such vectors comprise a said arranged sequence of at least 1) a first insertable DNA fragment; 2) a selectable marker gene; and 3) a second insertable DNA fragment.

The first and second insertable DNA fragments are each at least about 200 nucleotides in length and have nucleotide sequences which are homologous to portions of the genomic DNA of species of the genus Pichia. The various components of the integrative vector are serially arranged forming a linear fragment of DNA such that the expression cassette and the selectable marker gene are positioned between the 3′ end of the first insertable DNA fragment and the 5′ end of the second insertable DNA fragment. The first and second insertable DNA fragments are oriented with respect to one another in the serially arranged linear fragment as they are so oriented in the parent genome.

Nucleotide sequences useful as the first and second insertable DNA fragments are nucleotide sequences which are homologous with separate portions of the native genomic site at which genomic modifications is to occur. Thus, for example, if genomic modification is to occur at the locus of the alcohol oxidase gene, the first and second insertable DNA fragments employed will be sequences homologous with separate portions of the alcohol oxidase gene locus. For genomic modification in accordance with the present invention to occur, the two insertable DNA fragments must be oriented with respect to one another in the linear fragment in the same relative orientation as they exist in the parent genome. Examples of nucleotide sequences which could be used as first and second insertable DNA fragments are nucleotide sequences selected from the group consisting of the alcohol oxidase (AOX1) gene, dihydroxyacetone synthase (DHAS1) gene, p40 gene and HIS4 gene. The AOX1 gene, DHAS1 gene, p40 gene and HIS4 gene are contained in EP-A-0183071 incorporated herein by reference.

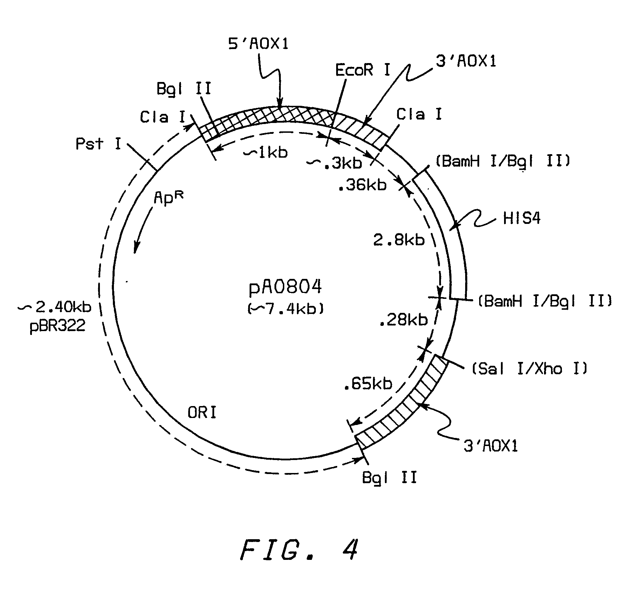

The first insertable DNA fragment may contain an operable regulatory region which may comprise the regulatory region utilized in the expression cassette. The use of the first insertable DNA fragment as the regulatory region for an expression cassette is a preferred embodiment of this invention. Figure 4 provides a diagram of a vector utilizing the first insertable DNA fragment as a regulatory region for a cassette.

Optionally as shown in Figure 4 an insertion site or sites and a 3′ termination sequence may be placed immediately 3′ to the first insertable DNA fragment. This conformation of the linear site-specific integrative vector has the additional advantage of providing a ready site for insertion of a structural gene without necessitating the addition of a compatible 3′ termination sequence.

It is also necessary to include at least one selectable marker gene in the DNA used to transform the host strain. This facilitates selection and isolation of those organisms which have incorporated the transforming DNA. The marker gene confers a phenotypic trait to the transformed organism which the host did not have, e.g., restoration of the ability to produce a specific amino acid where the untransformed host strain has a defect in the specific amino acid biosynthetic pathway or resistance to antibiotics and the like.

Exemplary selectable marker genes may be selected from the group consisting of the HIS4 gene and the ARG4 gene from Pichia pastoris and Saccharomyces cerevisiae, the invertase gene (SUC2) from Saccharomyces cerevisiae, or the G418 phosphotransferase gene from the E. coli transposable elements Tn601 or Tn903.

Those of skill in the art recognize that additional DNA sequences can also be incorporated into the vectors employed in the practice of the present invention, such as for example, bacterial plasmid DNA, bacteriophage DNA, and the like. Such sequences enable the amplification and maintenance of these vectors in bacterial hosts.

If the first insertable DNA fragment does not contain a regulatory region, a suitable regulatory region will need to be inserted operably linked to the structural gene, in order to provide an operable expression cassette. Similarly if no 3′ termination sequence is provided at the insertion site to complete the expression cassette, a 3′ termination sequence will have to be operably linked to the structural gene to be inserted.

Those skilled in the art are aware of numerous regulatory regions which have been characterized and could be employed in conjunction with methylotrophic yeasts. Exemplary regulatory regions include but are not limited to yeast regulatory regions selected from the group consisting of acid phosphatase, galactokinase, alcohol dehydrogenase, cytochrome c, alpha-mating factor and glyceraldehyde 3-phosphate dehydrogenase regulatory regions isolated from Saccharomyces cerevisiae; the primary alcohol oxidase (AOX1), dihydroxyacetone synthase (DAS1), the p40 regulatory regions, and the HIS4 regulatory region derived from Pichia pastoris and the like. Presently preferred regulatory regions employed in the practice of the present invention are those characterized by their ability to respond to methanol-containing media, such regulatory regions selected from the group consisting of AOX1, DHAS1, p40 and disclosed in co-pending application serial number 780,102.

The most preferred regulatory region for the practice of this invention is the AOX1 regulatory region.

3′ termination sequences may be utilized in the expression cassette or be part of the vector as discussed above. 3′ termination sequences may function to terminate, polyadenylate and/or stabilize the messenger RNA coded for by the structural gene when operably linked to a gene. A few examples of

8

illustrative sources for 3' termination sequences for the practice of this invention include but are not limited to the Saccharomyces cerevisiae, Hansenula polymorpha, and Pichia 3' termination sequences. Preferred are those derived from Pichia pastoris such as those selected from the group consisting of the 3' termination sequences of AOX1 gene, DHAS1 gene, p40 gene and HIS4 gene. And particularly preferred is the 3' termination sequence of the AOX1 gene.

For the practice of the current invention it is currently preferred to use linear transformation vectors such as the BglII fragments of the constructs shown in Figure 4.

The insertion of the preS₂ structural gene into suitable vectors may be accomplished by any suitable technique which cleaves the vector chosen at an appropriate site or sites and results in at least one operable expression cassette containing the preS₂ structural gene being present in the vector.

Ligation of preS₂ structural gene may be accomplished by any appropriate ligation technique such as utilizing T4 DNA ligase.

The initial selection, propagation, and optional amplification of the ligation mixture of the preS₂ structural gene and a vector is preferably performed by transforming the mixture into a bacterial host such as E. coli.

Suitable transformation techniques for E. coli are well known in the art. Additionally, selection markers and bacterial origins of replication necessary for the maintenance of a vector in a bacterial host are also well known in the art. The isolation and/or purification of the desired plasmid containing the preS₂ structural gene in an expression system may be accomplished by any suitable means for the separation of plasmid DNA from the host DNA.

Similarly the vectors formed by ligation may be tested preferably after propagation to verify the presence of the preS₂ gene and its operable linkage to a regulatory region and a 3' termination sequence. This may be accomplished by a variety of techniques including but not limited to endonuclease digestion, gel electrophoresis, or endonuclease digestion-Southern hybridization.

Transformation of plasmids or linear vectors into yeast hosts may be accomplished by suitable transformation techniques including but not limited to those taught by Hinnen et al, Proc. Natl. Acad. Sci. 75, (1978) 1929; Ito et al, J. Bacteriol 153, (1983) 163; Cregg et al Mol. Cell Biol. 5 (1985) pg. 3376; or Sreekrishna et al, Gene, 59 (1987) pg. 115. Preferable for the practice of this invention is the transformation technique of Cregg. It is desirable for the practice of this invention to utilize an excess of linear vectors and select for multiple insertions by Southern hybridization.

The yeast host for transformation may be any suitable methylotrophic yeast. Methylotrophic yeast include but are not limited to yeast capable of growth on methanol selected from the genera consisting of Hansenula, Candida, Kloeckera, Pichia, Saccharomyces, Torulopsis and Rhodotorula. A list of specific species which are exemplary of this class of yeasts may be found in C. Anthony, The Biochemistry of Methylotrophs, 269 (1982). Presently preferred are methylotrophic yeasts of the genus Pichia such as the auxotrophic Pichia pastoris GS115 (NRR Y-15851). Auxotrophic methylotrophic yeasts are also advantageous to the practice of this invention for their ease of selection. It is recognized that wild type methylotrophic yeast strains may be employed with equal success if a suitable transforming marker gene is selected, such as the use of SUC2 to transform Pichia pastoris to a strain capable of growth on sucrose, or an antibiotic resistance marker is employed, such as G418$^R$gene.

Transformed methylotrophic yeast cells can be selected by using appropriate techniques including but not limited to culturing previously auxotrophic cells after transformation in the absence of a biochemical product required (due to the cell's auxotrophy), selection by the detection of a new phenotype ("methanol slow"), or culturing in the presence of an antibiotic which is toxic to the yeast in the absence of a resistance gene contained in the transformant.

Isolated transformed methylotrophic yeast cells are cultured by appropriate fermentation techniques such as shake flask fermentation, high density fermentation or the techniques disclosed by Cregg et al, High-Level Expression and Efficient Assembly of Hepatitis B Surface Antigen in the Methylotrophic Yeast, Pichia Pastoris, 5 Bio/Technology 479 (1987).

Expression may be accomplished by methods appropriate to the regulatory region employed. Preferably if methanol responsive regulatory regions are utilized, the indication of expression may be accomplished by exposing the transformed cells in a nutrient media to an appropriate alcohol for the regulatory region employed.

The antigenic particles may be recovered in a crude form by lysing transformed cells which have been induced for a sufficient period, using standard techniques such as bead milling, followed by centrifugation sufficient to remove cellular debris. Those of skill in the art are aware of numerous methods available for the extraction of a heterologous protein from unicellular host organisms which could be substituted for the general extraction technique above or for futher purification. The preferred method of purification for the practice of this invention involves carrying out the lysis of the transformed cells in the presence of buffered chaotrophic compounds, precipitating lipids and contaminate proteins from the supernatant obtained from the lysis, diafiltering the supernatant, treating the retentate with silica, washing contaminate proteins from the silica-absorbed hepatitis B surface antigen with a buffer having a pH in the range of 6-8, eluting the antigen from the silica with a buffered elutant having a pH in the range of 9.5 to 11 containing from 0.5 to 8 molarity of urea, subjecting the antigen containing fractions so obtained to gel filtration and thereafter subjecting the antigen containing fraction to anionic exchange chromotography.

The following non-limiting examples are provided to further illustrate the practice of this invention.

# Examples

General information pertinent to the Examples:

Pichia pastoris GS115 (his4) NRRL Y-15851 was the host yeast strain used in these examples.

| Media | | |
|---|---|---|
| YPD, 1 liter | 10g | yeast extract |
| | 20g | peptone |
| | 20g | dextrose |
| LB broth, 1 liter | 5.0g | yeast extract (Difco) |
| | 10.0g | tryptone (Difco) |
| | 5.0g | NaCl |

# Example I

# Construction of Vector pTB04

"Plasmid AM6 is a derivative of the HBV genome shown in Figure 1, wherein the pBR 322 plasmid is inserted at the BamHI siteat position 26."

The vector pTB04 contains the gene encoding the 281 amino acid preS$_2$ form of the hepatitis B surface antigen. The gene was constructed in three segments: the C-terminal 75% of the structural gene, an N-terminal linker encoding 13 amino acids, and the remaining central portion. The plasmid AM6 containing the preS$_2$ gene, adw seratype, was the source of the C-terminal portion and the middle portion of the preS$_2$ gene used here. The sequence is disclosed in Valenzuela et al., ICN-UCLA Symposia on Animal Virus Genetics, p. 57-70 (1980), with the following modification:

$$1$$

native sequence            ATG-CAG-TGG-AAT-TCC

mutagenized sequence      ATG-CAG-TGG-AAC-TCC

## A. C-terminal portion of preS$_2$

(All restriction enzymes were obtained from Boehringer Mannheim, and used according to manufacturer's instructions).

The C-terminal portion was isolated from the plasmid AM6 (Figure 1) by digestion with DraI, which cuts the HBV genome at two places, one of which is at the codon for the last amino acid of the surface antigen. The ends were dephosphorylated by treatment with calf intestinal alkaline phosphatase in a 30μl reaction

volume (1U enzyme at 37°C for 1 hour in 50mM Tris•HCl, pH 9.0, 1mM MgCl₂, 100μM ZnCl₂, 1mM spermidine). The entire digest was phenol extracted and ethanol precipitated (Maniatis et al.). An octameric Stul linker (AAGGCCTT) was synthesized by a DNA synthesizer from Applied Biosystems Model 380A using cyanoethylophosphoramidite chemistry. 1μg of Stul linkers was dissolved in distilled water. A 10ng aliquot was removed and labeled with phosphate in a 50μl total volume containing 70mM Tris•HCl, pH 7.6, 10mM MgCl₂, 5mM dithiothreitol, 1mM ATP and 10 units of polynucleotide kinase for 30 minutes at 37°C. The linkers were heated to 90°C to terminate the enzymatic reaction, and slow-cooled to room temperature to facilitate double stranded DNA formation. The Stul linkers were added to the Dral digest above and ligated with T4 ligase as follows. The reaction occurred in a 10μl volume containing 6.6 M Tris•Cl, pH 7.6, 5mM MgCl₂, 5mM dithiothreitol, 1mM ATP and 1 Weiss unit of T4 ligase for 1 hour at 23°C. The ligation reaction was terminated by phenol extraction followed by ethanol precipitation. A Stul restriction digest was then performed with >50 U of enzyme overnight to remove multimers of the Stul linker. The combination of Dral digestion and Stul linkers restored the translation stop codon removed by Dral digestion.

The Stul-linkered Dral fragments were digested with Xbal, which yielded the desired Stul/Xbal fragment of approximately 600 bp; it was isolated from a 0.8% preparative agarose gel. This fragment contained the C-terminal 75% of the gene and was cloned into the vector pYM4 (Figure 2) which had been digested with Xbal and Stul, and dephosporylated as above. (pYM4 can be obtained from plasmid pYM30 by digesting pYM30 with Clal and re-ligating the ends. pYM30 is available in an E. coli host deposited at the Northern Regional Research Center, United States Department of Agriculture, Peoria, Illinois, accession number NRRL B-15890). The 5.7 Kb restriction fragment of pYM4 was isolated from a 0.8% preparative agarose gel. Vector pYM4 was employed solely for its convenient restriction sites. 50ng of the vector and 500 ng of the insert were ligated at 23°C for 1 hour in 50 mM Tris HC1 pH 7.4, 10 mM MgCl₂, 10 mM dithiotreitol, 1 mM spermidine, 1mM ATP with 1 Weiss Unit of T4 ligase in a 10μl volume. The ligation reaction was used directly to transform competent MC1061 cells (E. coli) to ampicillin resistance. E. coli strain MC1061 is available at the Northern Regional Research Center, United States Department of Agriculture, Peoria, Illinois, accession number NRRL-18016. MC1061 has the following genotype: F(-), ara D139 delta (lacIPOZY) X74 galk galU hsr hsm(+) rpsL delta (araABOIC leu) 7697. MC1061 was rendered competent for transformation in the following manner. A mid-log culture (50 ml) of E. coli MC1061 was harvested by centrifugation in a Damon IEC DPR600 centrifuge at 3,000 rpm for 5 min. at 4°C and washed in 10 mM NCl. The culture was resuspended in 25ml of 50 mM CaCl₂ for 30 minutes at 0°C. The cells were centrifuged as above and resuspended in 2ml of 50 mM CaCl₂. For transformation, the ligation reaction was added to 100μl of the competent cell suspension and incubated at 0°C on ice for 15 minutes, heat shocked at 37°C for 5 minutes and incubated at 23°C for 5 minutes. The cells were plated directly onto LB agar plates containing 50μg/ml ampicillin. The plates were incubated at 37°C for 10-16 hours. The resulting colonies were harvested and characterized by restriction digestion. Cells were grown in 5ml of L-broth containing 50μg/ml ampicillin for 5 hr. at 37°C and DNA was prepared by the method of Birnboim and Doly [Nucleic Acids Research 7: 1513 (1979)]. The minipreps were digested with BamHI and Xbal. Cultures yielding a 1.5 Kb Xba/Bam fragment were deemed to have the insert and a large scale DNA prep of one culture was purified as above, followed by banding on a cesium chloride-ethidium bromide gradient. This clone was called pHS1.

The plasmid pHS1 was digested with Stul, dephosphorylated as above, phenol extracted and ethanol precipitated. EcoRI linkers (GGAATTCC) synthesized as above were phosphorylated, self annealed, and ligated to this blunt ended DNA. Excess linkers were removed by overnight EcoRI digestion and the DNA was subsequently digested with Xbal following phenol extraction and ethanol precipitation. A doublet containing the 600 bp Xbal-EcoRI fragment of interest and a vector fragment of 582 bp (Xbal-EcoRI) was isolated by 1.0% preparative gel electrophoresis. These fragments (500 ng) were ligated into Xbal-EcoRI-digested and dephosphorylated pUC18 (50 ng) as described above and used to transform MC1061 to ampicillin resistance as described above. Restriction digests of miniprep DNA were used to determine which of the two fragments had been cloned. The undesirable fragment had a Clal site such that a Clal/Xbal double digest would yield fragments of approximately 560 bp + 2400 bp, whereas the correct fragment yielded a linear 3 Kb fragment upon Clal digestion. Candidates were digested with EcoRI and Xbal to yield fragments of 600 bp + 2400 bp. One such clone was purified on large scale and termed pHS2-B. This contains an EcoRI site after the last codon at the C-terminal region of the complete preS₂ gene.

## B. Middle Portion of preS₂ Gene

The middle portion of the preS₂ gene was cloned as follows. The plasmid AM6 was digested with Xbal

and BamHI and a fragment of 250 bp was isolated from a 0.8% preparative agarose gel. This fragment (50 ng) was ligated as described above to 5 ng of pUC18 digested with XbaI and BamHI and dephosphorylated. The ligation reaction was used to transform E. coli strain MC1061 to ampicillin resistance as above. Minipreps were digested with BamHI and those containing a 2.7 Kb linear fragment were chosen. One isolate was grown on large scale and DNA was isolated and purified as described. This clone is called pPS1. The clone was cut with EcoRI and BamHI and the vector band isolated and purified by 0.8% preparative agarose gel electrophoresis. To this vector was ligated the following kinased double stranded oligonucleotide synthesized as above:

```
               EcoRI    PstI   BamHI
         5' AATTCAATCCGTCTGCAG 3'

            3' GTTAGGCAGACGTCCTAG 5'
```

The ligation reaction was used to transform E. coli MC1061 to ampicillin resistance. Minipreps were characterized by PstI digestion. One clone containing a 250 bp PstI fragment was chosen, a large scale DNA prep was performed, and the plasmid pPS2 was isolated.


## C. N-terminal Portion of preS₂ Gene

The N-terminal region encompassing the EcoRI linker and the coding sequences for the first thirteen amino acids was generated from a synthetic oligonucleotide containing the following sequence synthesized as above:

```
      HindIII EcoRI                                    PstI
       5' AGCTTGAATTCATGCAGTGGAACTCCACTGCCTTCCACCAAACTCTGCA 3'

          3' ACTTAAGTACGTCACCTTGAGGTGACGGAAGGTGGTTTGAG 5'
```

This fragment contained HindIII and PstI ends as well as an EcoRI sequence preceding the ATG. This sequence wsa cloned into HindIII- and PstI-digested and dephosphorylated pUC18 by ligating a ten-fold excess of the oligonucleotide into the vector and characterizing the transformants by the presence of a small EcoRI site (~ 75 bp). Such a clone was designated pTBO-2A.

The middle portion of the gene was added by digesting the vector pTB0-2A with PstI and XbaI; the insert was the 250 bp Pst-Xba fragment from pPS2. Transformants were characterized by the presence of a >300 bp EcoRI fragment as well as 290 bp XbaI/HindIII fragment. The correct isolate was termed pTB0-3. The complete preS₂ gene was achieved by inserting the HindIII/XbaI fragment from pTB0-3 into XbaI/HindIII-digested pHS2B. Ampicillin resistant transformants were characterized as above by the presence of a 825 bp EcoRI fragment. This construct was termed pTB04.


## Example II


## Construction of Vector pTB05A

The pA0804 vector is available in an E. coli host from the Northern Regional Research Center of the United States Department of Agriculture, Peoria, Illinois, accession number NRRL B-18114.pA0804 is recovered by isolating the plasmid DNA, digesting with EcoRI, gel electrophoresing to recover the ~ 7.5 KB fragment, which is linear pA0804 cut at its unique EcoRI site.

A vector containing the gene coding for preS₂ was constructed from vectors pA0804 and pTB04 (pTB04 was derived in Example I). 2µg of pA0804 was digested with EcoRI as before and treated with alkaline phosphatase in a 30µl reaction volume (1 U enzyme at 37°C for 1 hour in 50mM Tris•HCl pH 9.0, 1mM MgCl₂, 100 µM ZnCl₂, 1mM spermidine). pTB04 was subjected to EcoRI digestion and an 825 bp fragment encoding the preS₂ gene was released. This fragment was purified using preparative agarose gel electrophoresis using 0.8% agarose. 500ng of the fragment and 50ng of pA0804 were ligated using methods described in Example I. The resulting vector was used to transform MC1061 to ampicillin resistance using the method described in Example I. The DNA was isolated using the method of Birnboim and Doly [Nucleic Acids Research 7:1513 (1979)] and characterized by digestion with PstI. A clone containing a 2.1 Kb PstI fragment was determined to have the insert in the correct orientation and was designated pTB05A.

## Example III

### Development of Multicopy preS₂ Strain GS115/pTB05A(S10) and Single Copy Strain GS115/pTB05A

The multicopy strain GS115/pTB05A(S10) was generated by purifying the 5.9 Kb BglII fragment from pTB05A on a 0.8% preparative agarose gel. 10μg of the fragment was self-ligated overnight using conditions described in Example I. Aliquots of the ligation reaction were monitored for the presence of high molecular weight material on a 0.6% agarose gel. This was used to transform Pichia pastoris GS115 using the spheroplast transformation technique described by Cregg et al., Bio/Technology 5:479-485 (1987). Transformants were regenerated on minimal media and screened for the appropriate mutant phenotype in the following manner. Transformants were pooled by scraping the surface of the plate in the presence of sterile distilled water and sonicated at low output for 15 seconds. They were subsequently diluted to an $A_{600}$ = 0.1 and plated at dilutions of $10^{-3}$ and $10^{-4}$ in duplicate onto minimal plates containing glycerol as the carbon source, and incubated at 30°C for 2-3 days. They were then replica-plated onto minimal plates to which 100μl of methanol was added in the vapor phase. After a 24-hour incubation at 30°C, it was apparent that 10-20% of the transformants were growing more slowly on methanol than the rest of the transformants. Ten of these slow growing colonies were then selected for further analysis. They were picked from the minimal plate containing glycerol, grown in shake flasks as described in Example V, and assayed for 22 nm - like particle activity as described in Example VIII. One transformant having a four-fold elevated activity was further characterized by Southern blot analysis (Maniatis et al.). A BglII digest of the multicopy transformant was transferred to nitrocellulose and the filter was probed with the nick-translated HIS4 specific probe, pYM4 (pYM4 is described in Example I). The blot contained two hybridizing bands: one from the genomic HIS4 locus and one from the expression vector. A ratio of the intensity of the bands derived from the expression cassettes in the multicopy strain relative to a single copy, normalized to the intensity of the HIS4 genomic bands, yielded a total of 4 copies per cell.

The single copy strain GS115/pTB05A was generated in the same manner, with the omission of the fragment self-ligation reaction.

## Example IV

### Yeast DNA Miniprep

$10^{4}$ cells/ml were grown in 5ml YPD at 30°C overnight and then pelleted using a Damon IEC DPR600 clinical centrifuge at 3,000 rpm for 5 minutes. The pellet was resuspended in 0.5 ml of 1M sorbitol, 0.1ml 0.5M EDTA, pH 7.5 and the sample transferred to a 1.5ml microfuge tube. 0.02ml of 2.5 mg/ml Zymolyase 60,000 (Miles Laboratories) was added, and the sample was incubated at 37°C for 60 minutes. The cells were pelleted using the microfuge for 1 minute at high speed, and resuspended in 0.5 ml of 50mM Tris•HCl, pH 7.4 and 20mM EDTA. 0.05 ml of 10% SDS was added, the sample mixed, and incubated at 65°C for 30 minutes. 0.2 ml of 5M potassium acetate was added and the sample was incubated on ice for 60 minutes. The sample was again spun in a microfuge at high speed for 5 minutes.

The supernatant was transferred to a fresh 1.5ml microfuge tube and 1 volume of isopropanol at room temperature was added. The sample was mixed and allowed to sit at room temperature for 5 minutes, then spun very briefly (10 seconds) in a microfuge at high speed. The supernatant was poured off and the pellet air dried. After resuspending the pellet in 0.3 ml of 10mM Tris•HCl, pH 7.4 and 1 mM EDTA, 15µl of a 1 mg/ml solution of pancreatic RNase, was added, and the sample was incubated at 37°C for 30 minutes. 0.03ml of 3M sodium acetate was added, the sample mixed, and 0.2ml of isopropanol added. The sample was spun in a microfuge at high speed to pellet the DNA. The supernatant was then poured off, the pellet dried and resuspended in 0.1-0.3ml of 10mM Tris•HCl, pH 7.4 and 1mM EDTA. (Note: Before using the DNA in a restriction digest, it may be necessary to spin the solution for 15 minutes at high speed in the microfuge to remove any insoluble material which may inhibit the digestion).

## Example V

### Shake Flask Expression Studies

Prior to fermentation, all strains were grown in shake flasks as follows to ascertain expression levels. Routinely, a transformant was seeded into 0.67% yeast nitrogen base containing 2-5% glycerol and grown overnight at 30°C into middle to late log phase. The cells were then collected to centrifugation using a Damon IEC DPR600 clinical centrifuge at 3,000 rpm for 5 minutes. The pellet was washed in sterile water twice, then seeded at a density of 0.5 $A_{600}$ units/ml into 0.67% YNB containing 1% methanol and grown for 4-6 days at 30°C with moderate shaking. At various times, aliquots of 50 $A_{600}$ units were removed and stored at -20°C. Protein extracts were prepared from these aliquots to be used for an AUSRIA™ and Western blot analysis (see Examples VIII and VII, respectively). Aliquots of cells (100 $A_{600}$ units) were transferred to 13 x 100mm borosilicate culture tubes and washed twice by centrifugation in a Sorvall Model RC-5B at 12,000 rpm, 4°C with 20 volumes of lysing buffer [0.5 M NaCl, 0.1% Triton X-100 (w/v), 1M phenylmethylsufonyl fluoride and 10mM sodium phosphate, pH 7.5]. Cell samples were resuspended with 0.5 grams of acid-washed glass beads (0.5mm) plus 0.35ml of lysing buffer, and agitated for eight, one-minute intervals at maximum speed using a vortex mixer. Between intervals, the mixture was cooled on ice for at least one minute. After lysing was completed, the solution of broken cells was removed, the glass beads were washed with 0.35ml of lysing buffer, and the two solutions combined and centrifuged using the Sorvall RC-5B at 13,000 rpm, 4°C to remove cellular debris. Protein samples were then assayed by AUSRIA™ and by Western blot analysis (see Table I). Protein concentration was determined by the Lowry method after TCA precipitation.

Table I

| Analysis of preS₂ Expression | | | |
|---|---|---|---|
| | Strain | % Protein AUSTRIA™ | % Protein Western |
| 1. | GS115/pTB05A | 0.11% | 0.23% |
| 2. | GS115/pTB05A(S10) | 0.31% | 0.63% |

## Example VI

### Fermentation Expression Studies

Fermentations were performed as follows. Five hundred ml of yeast Nitrogen Base (YNB) +2% glycerol in a Fernbach Flask was inoculated from a seed culture or a minimal glucose plate of the culture. (Plates may be maintained by monthly passage with no detectable strain deterioration). After one day of shaking at 200 rpm and 30°C, the inoculum was seeded into 7.5-liter minimal medium (Table II) containing 480g glycerol, 40 mg biotin, and 40ml trace salts solution (Table III). The fermentor was maintained at 30°C and pH 5.5 while the culture grew in batch mode until the glycerol was exhausted (about 24 hours). The pH was controlled by the addition of $NH_3$ gas. Glycerol exhaustion was noted by a sharp decline in the $CO_2$ evolution and a sharp rise in the dissolved oxygen (or decrease in oxygen uptake rate). A methanol feed was initiated at 18 ml/hr to bring the fermentor level up to ~0.5% MeOH, and maintained at this level. The flow rate was adjusted based on the actual methanol consumption rate. Twenty ml aliquots of trace salts were added at approximately two day intervals to maintain the methanol consumption rate. The level of HBsAg increased for about three days on the methanol feed.

Table II

| Medium Composition (7.5-Liter) | |
|---|---|
| 480 g | glycerol |
| 40 mg | biotin |
| 134 ml | $H_3PO_4$ (85%) |
| 5.8 g | $CaSO_4 \cdot 2H_2O$ |
| 92 g | $H_2SO_4$ |
| 75 g | $MgSO_4 \cdot 7H_2O$ |
| 21 g | KOH |

Table III

| $IM_1$ Trace Salts Solution | |
|---|---|
| Cupric Sulfate $\cdot 5H_2O$ | 0.06 g/$\ell$ |
| Potassium Iodide | 0.08 " |
| Manganese Sulfate $\cdot H_2O$ | 0.30 " |
| Sodium Molybdate | 0.20 " |
| Boric Acid | 0.02 " |
| Zinc Sulfate $\cdot H_2O$ | 2.00 " |
| Ferric Chloride $\cdot H_2O$ | 4.8 " |
| Sulfuric Acid | 5.00 ml/liter |

Table IV shows the AUSRIA™ results as described in Example VIII on extracts prepared from strains grown in the fermentor.

Table IV

| Strain | Volumetric yield (mg/L) via AUSTRIA™ |
|---|---|
| GS115/pTB05A | 9 |
| GS115/pTB05A(S10) | 60 |

## Example VII

## Detection and Confirmation of the Presence of preS$_2$

Western Blots were performed (Maniatis et al.) on proteins recovered from lysed Pichia cells as discussed in Example V. The antibody used was specific for HBsAg, obtained from Cal Biochem, Lot# 702106, at a 1:1000 dilution.

15

In addition, SDS/PAGE analysis and silver staining of a partially purified protein preparation indicated the presence of preS$_2$ polypeptide, p31.

## Example VIII

### AUSRIA™ RIA Protocol

The Abbott AUSRIA™ assay kit was used to measure the amount of HBsAg synthesized by the Pichia production system. The antibody contained in the kit binds to HBsAg particles, not HBsAg monomers. All dilutions were made in 1,0% BSA, 0.02% Na Azide in phosphate buffered saline, pH 7.4. The procedure followed was essentially as outlined in the kit instructions. The standard curve was prepared as follows.

| Tube # | ng. in assay | Buffer($\mu$l) | Positive Control ($\mu$l) |
|---|---|---|---|
| 1-4 | None NSB | none | 200 buffer only |
| 5-6 | 0.1 | 195 | 5 |
| 7-8 | 0.2 | 190 | 10 |
| 9-10 | 0.5 | 175 | 25 |
| 11-12 | 1.0 | 150 | 50 |
| 13-14 | 2.0 | 100 | 100 |
| 15-16 | 3.0 | 50 | 150 |
| 17-18 | 4.0 | none | 200 |

The wells of the microtiter dish were labeled as follows.

| | AA | BB | CC | DD |
|---|---|---|---|---|
| 1 | 1 | 2 | 3 | 4 |
| 2 | 5 | 6 | 7 | 8 |
| 3 | 9 | 10 | 11 | 12 |
| 4 | 13 | 14 | 15 | 16 |
| 5 | 17 | 18 | 19 | 20 and so on... |

The beads were first added to each well, followed by the buffer, and finally the standard (positive control) or the diluted sample. Unknowns were diluted to obtain signals within the range of the standard curve. Estimates of sample concentrations in mg/ml were typically divided by 0.02 to obtain the dilution to be used. Usually 100$\mu$l of the sample was added to the well containing 100$\mu$l of buffer. The wells were covered and the tray gently tapped against the bench top. The samples were then incubated overnight at room temperature to attain maximum binding efficiency. The next morning each well was washed 4 times with deionized water using the Pentawash system provided by Abbott Labs. 200$\mu$l of the $^{125}$I anti-HBs were added to each well, the tray was gently tapped, and then incubated in a 45°C water bath for 1 hr. The beads were washed as before and counted. Concentrations of unknowns were determined from the standard curve.

## Claims

1. A process for the enhanced production of antigenic HBV particles comprising

a) transforming a methylotrophic yeast with at least one vector having at least one expression cassette containing a structural gene for preS$_2$, operably linked to a regulatory region and a 3′ termination sequence; and thereafter

16

b) culturing the resulting transformed yeast strain under suitable conditions to obtain the production of said HBsAg preS$_2$ protein.

2. The process of claim 1, wherein said vector is selected from plasmids or linear, integrative site-specific vectors.

3. The process of claim 2, wherein said linear integrative site-specific vector contains the following serial arrangement:

a) a first insertable DNA fragment,

b) a marker gene, and at least one expression cassette containing a structural gene for preS$_2$, operably linked to a regulatory region and a 3′ termination sequence, and

c) a second insertable DNA fragment;

wherein the order of the marker gene and cassette of component (b) may be interchanged.

4. The process of claim 3, wherein the first insertable DNA fragment and the second insertable DNA fragment are derived from the DNA sequence of a gene isolated from Pichia pastoris and selected from AOX1, p40, DHAS and HIS4.

5. The process of claim 3, wherein said expression cassette comprises

a) a regulatory region selected from AOX1, p40, DHAS and HIS4 isolated from Pichia pastoris, acid phosphatase, galactosidase, alcohol dehydrogenase, cytochrome c, alpha-mating factor and glyceraldehyde 3-phosphate dehydrogenase isolated from Saccharomyces cerevisiae operably linked to

b) a structural gene for preS$_2$, operably linked to

c) a 3′ termination sequence from Pichia pastoris selected from the 3′ termination sequences isolated from the AOX1 gene, p40 gene, DHAS gene and HIS4 gene.

6. The process of claim 3, wherein said marker gene is selected from HIS4 and ARG4, isolated from Pichia pastoris, SUC2 isolated from Saccharomyces cerevisiae and G418$^R$ gene of Tn903 and Tn601.

7. The process of claim 3, wherein said vector comprises

a) a first insertable DNA fragment which is about one kilobase of the 5′ AOX1 regulatory region isolated from Pichia pastoris operably linked to

b) a structural gene for preS$_2$, operably linked to

c) the 3′ termination sequence of AOX1 isolated from Pichia pastoris ligated to

d) a marker gene which is HIS4 isolated from Pichia pastoris ligated to

e) a second insertable DNA fragment which is about 0.65 kilobases of the 3′ AOX1 termination sequence.

8. A linear integrative site-specific vector comprising the following serial arrangement

a) a first insertable DNA fragment,

b) a marker gene and at least one expression cassette containing a structural gene for preS$_2$, operably linked to a regulatory region and 3′ termination sequence, and

c) a second insertable DNA fragment;

wherein the order of the marker gene and cassette of component (b) may be interchanged.

9. The vector of claim 8, wherein said vector is as defined in any of claims 4 to 7.

10. Methylotrophic yeast transformed with at least one vector containing at least one expression cassette comprising a regulatory region operably linked to a structural gene for preS$_2$, operably linked to a 3′ termination sequence.

11. The methylotrophic yeast of claim 10, wherein the yeast is Pichia pastoris.

12. The methylotrophic yeast of claim 11, wherein the yeast is Pichia pastoris strain GS115.

13. The yeast of any of claims 10 to 12, wherein said yeast is transformed with at least one linear integrative site-specific vector as defined in any of claims 3 to 9.

14. Pichia pastoris GS115/pTB05A.

15. Pichia pastoris GS115 transformed as in claim 13, wherein said GS115 is transformed with more than one copy of said linear integrative site-specific vector.

16. Pichia pastoris GS115/pTB05A (S10).

FIG. 1

## RESTRICTION SITES IN AM6 (HBV)

| | |
|---|---|
| Ava I | 1461 |
| Bam HI | 1398 |
| Bam HI | 26 |
| Bgl II | 1982 |
| Bgl II | 2403 |
| Bgl II | 2427 |
| Bst E II | 2819 |
| Dra I | 829 |
| Dra I | 2180 |
| Hae II | 1435 |
| Hinc II | 215 |
| Hinc II | 959 |
| Hinc II | 1680 |
| Hinc II | 2584 |
| Hinc II | 3115 |
| Hpa II | 1303 |
| Hpa II | 1568 |
| Hpa II | 2328 |
| Pst I | 21 |
| Stu I | 965 |
| Stu I | 1110 |
| Stu I | 1697 |
| Xba I | 245 |

FIG. 2

[Cla I (23)]
Hind III (~213)
Eco RV (~369)
Eco RI
[Bam/Bgl]
(6819) Pvu I
Xba I (~757)
(6693) Pst I
Kpn I (~834)
Bst X I (~1309)
[Taq]    184 bp    [Taq]
Kpn I (~1759)
Sal I (~1809)
109    50   25
Bgl II    Nru I
Stu I (~2059)
pYM10
7.2 kb
HIS4
Pvu II
(5150)
[Bam HI (~2959)]
Pvu II (~3159)
Ava I
(4509)
[Bam/Bgl]
Nru I
(4056)
Sal I
(3735)

FIG. 3

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | EP - A1 - 0 226 846 (PHILLIPS PETROLEUM COMPANY) * Claims 1-4,9,10 * | 1-4,8, 10,11 | C 12 N 15/00 C 12 P 21/00 C 12 N 1/16 //(C 12 N 1/16 C 12 R 1:84) |
| D,A | BIOTECHNOLOGY, vol. 5, no. 5, May 1987 (New York) J.M.CREGG et al. "High-Level Expression and Efficient Assembly of Hepatits B Surface Antigen in the Methylotrophic Yeast, Pichia pastoris" pages 479-485 * Totality * | 1-4, 10-16 | |
| A | EP - A2 - 0 183 070 (PHILLIPS PETROLEUM COMPANY) * Abstract; claims 1,3 * | 1,10- 16 | |
| D,A | EP - A1 - 0 226 752 (PHILLIPS PETROLEUM COMPANY) * Abstract * | 1-3,8 | |
| D,A | EP - A2 - 0 183 071 (PHILLIPS PETROLEUM COMPANY) * Abstract * | 1 | TECHNICAL FIELDS SEARCHED (Int. Cl.4) C 12 N C 12 P |
| P,A | EP - A2 - 0 278 940 (SMITH KLINE-RIT SOCIETE ANONYME DITE) * Abstract; claims 6,7 * | 1 | |
| A | EP - A1 - 0 244 924 (MERCK & CO. INC.) * Abstract; claim 1 * | 1 | |

The present search report has been drawn up for all claims

| Place of search | Date of completion of the search | Examiner |
|---|---|---|
| VIENNA | 02-08-1989 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82